(19) Europäisches Patentamt
European Patent Office
Office européen des brevets

(11) Veröffentlichungsnummer: **0 014 965**
**B1**

(12) # EUROPÄISCHE PATENTSCHRIFT

(45) Veröffentlichungstag der Patentschrift: **24.10.84**

(51) Int. Cl.³: **G 01 N 33/76,** G 01 N 33/54

(21) Anmeldenummer: **80100755.0**

(22) Anmeldetag: **14.02.80**

(54) **Immunologisches diagnostisches Reagenz zur Bestimmung der Schwangerschaft, Verfahren zu dessen Herstellung sowie dessen Verwendung zur Bestimmung der Schwangerschaft.**

(30) Priorität: **16.02.79 US 12629**

(43) Veröffentlichungstag der Anmeldung: **03.09.80 Patentblatt 80/18**

(45) Bekanntmachung des Hinweises auf die Patenterteilung: **24.10.84 Patentblatt 84/43**

(84) Benannte Vertragsstaaten: **AT BE CH DE FR GB IT NL SE**

(56) Entgegenhaltungen:
**EP - A - 0 001 224**
**FR - A - 2 384 789**
**GB - A - 2 013 690**
**US - A - 3 992 514**
**US - A - 4 123 509**
**US - A - 4 138 214**

(73) Patentinhaber: **F. HOFFMANN-LA ROCHE & CO. Aktiengesellschaft, CH-4002 Basel (CH)**

(72) Erfinder: **Hansen, Hans John, Maple Street 45, Allendale, N.J. (US)**

(74) Vertreter: **Lederer, Franz, Dr. et al, Patentanwälte Dr. Franz Lederer Reiner F. Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein immunologisches diagnostisches Reagenz zur Bestimmung der Schwangerschaft mittels HCG-Bestimmung und Latex-Agglutination, ein Verfahren zur Herstellung dieses Reagenz sowie ein Verfahren zur Bestimmung der Schwangerschaft.

Die Verwendung von menschlichem Choriongonadotropin (nachfolgend HCG genannt) zur Herstellung von Latexreagenzien zur Bestimmung der Schwangerschaft ist längst bekannt. So wird z.B. im US-Patent Nr. 3 857 931, welches am 31. Dezember 1974 veröffentlicht wurde, die Herstellung und Verwendung solcher Reagenzien beschrieben. Im weiteren sind eine Reihe von Reagenzien zur Bestimmung der Schwangerschaft im Handel, welche dem Latex-HCG-Typ entsprechen. Ein ähnliches Reagenz wird auch in der französischen Patentschrift Nr. 2 234 789 beschrieben.

Die kommerziell erhältlichen Latex HCG Schwangerschaftstests benutzen in der Regel HCG in einer teilweise gereinigten Form, welches an einen serologisch inerten Latex Polymerträger gebunden ist. Bei Gebrauch des Tests wird ein Tropfen Urin einer Frau auf einen gereinigten Glasobjektträger gegeben, mit einem Tropfen Anti-HCG Serum vermischt und dann mit einem Tropfen einer wässrigen Suspension eines Latexträgers, an den HCG gebunden ist, vermischt. Nach wenigen Minuten wird der Objektträger daraufhin untersucht, ob eine Agglutination stattgefunden hat, was ein negatives Resultat bedeutet. Dies ist nur eine sehr allgemeine Angabe der Methode, denn es existieren Varianten sowohl bezüglich der Technik wie bezüglich der verwendeten Materialien.

Da eine Anzahl Schwangerschaftstests auf der HCG Latexbasis kommerzialisiert sind, ist in diesem kompetitiven Gebiet ein konstantes Bemühen, diese Tests zu verbessern, und zwar bezüglich der Sensibilität, der Materialkosten, der Herstellung sowie der Bequemlichkeit in der Handhabung und Verwendung. Bei der Suche nach mehr spezifischeren Tests wurde gefunden, dass durch die Auftrennung von HCG in seine α- und β-Kette und nachfolgendes Reinigen der β-Kette ein höchst spezifisches Reagenz hergestellt werden kann [Morgan and Canfield, Endocrinology, Vol. 88, p. 1045 (1971)].

Es gibt bekannte Methoden zur Reinigung der β-Kette von HCG, doch sind die bisher bekannten Methoden äusserst teuer und daher – obwohl ein Reagenz mit verbesserter Spezifität mit reiner β-Kette von HCG erhalten werden kann – ist die Kommerzialisierung als ein Test auf Latexbasis praktisch ausgeschlossen. Das einzige Produkt auf Latextasis, das sich im Markt befindet und das β-Kette Material verwendet, ist ein Produkt, welches β-Kette Antiserum und keine gereinigte β-Kette von HCG Antigen benutzt, was wohl auf die kostspielige Reinigung der β-Kette von HCG zurückzuführen ist.

Im Rahmen der vorliegenden Erfindung wurde nun eine Methode gefunden, wonach rohes HCG, wie es kommerziell erhältlich ist, ohne zusätzliche aufwendige Reinigung behandelt werden kann, um die β-Kette von HCG zu erhalten, welche – unerwarteterweise – immunologisch sehr reaktiv ist. Die β-Kette von HCG gemäss vorliegender Erfindung kann in einfacher Weise an immunologisch inerte Latexpartikel gebunden werden, wobei man ein Reagenz erhält, welches 2–4fach mehr sensitiv ist, als ähnliche bekannte Reagenzien, welche kommerziell erhältliches HCG verwenden. Die grosse Sensibilität des Reagenz gemäss vorliegender Erfindung ist beachtlich vorteilhaft, wenn man bedenkt, dass es von relativ unreinem HCG hergestellt werden kann, ohne aufwendige Reinigungstechniken, welche man bisher als notwendig erachtet hat bei der Verwendung der β-Kette von HCG.

Gegenstand der vorliegenden Erfindung ist demnach ein immunologisches diagnostisches Reagenz zur Bestimmung der Schwangerschaft mittels HCG-Bestimmung und Latex-Agglutination enthaltend diskrete Partikel eines immunologisch inerten Latex-Polymers mit einer Teilchengrösse von 0,01 bis 0,9 μm und einem spezifischen Gewicht von 1,01 bis 1,1, an die mit einem Carbodiimid-Kopplungsmittel bei einem pH von 5 bis 8 menschliches Choriongonadotropin (HCG) durch eine Amidbrücke kovalent gebunden worden ist, welches dadurch gekennzeichnet ist, dass man das HCG in Form seiner β-Kette einsetzt, wobei diese β-Kette durch ½ bis 2 stündiges Behandeln von teilweise gereinigtem HCG enthaltend 2000–5000 IE/mg HCG bei einem pH von 4–5,5 und einer Temperatur von 20–50 °C mit einem chaoptropischen Mittel, nämlich mit einer 7–10 molaren wässrigen Lösung von Harnstoff, einer 7–10 molaren wässrigen Lösung von Lithiumbromid, einer 4,5–6 molaren wässrigen Lösung von Guanidinhydrochlorid oder einer 2–3 molaren wässrigen Lösung von Ammoniumthiocyanat, anschliessende Einstellung des pH des Reaktionsgemisches auf 7,5 und Abtrennung des Gemisches HCG/chaotropisches Mittel durch Ionenaustausch und/oder weitere herkömmliche Methoden, gemäss welchen Moleküle auf Grund von Differenzen in der elektrostatischen Ladung getrennt werden können, von der α-Kette abgetrennt worden ist und wobei weder das teilweise gereinigte HCG noch die β-Kette davon weiter gereinigt worden sind. Die so erhaltene β-Kette von HCG zeigt eine hohe immunologische Sensitivität, obwohl sie immer noch eine grosse Menge von Proteinen, z.B. Urinproteinen, enthält, welche vom ungereinigten Ausgangsmaterial herstammen.

Die Erfindung umfasst auch ein Verfahren zur Herstellung dieses Reagenz gemäss Anspruch 5, sowie ein Verfahren zur Bestimmung der Schwangerschaft gemäss Anspruch 7.

Die β-Kette von HCG, welche gemäss der Methode der vorliegenden Erfindung erhalten wird, kann leicht in an sich bekannter Weise an immunologisch inerte Latexträgerpartikel gebunden werden. Vom erhaltenen Reagenz wurde gefunden, dass es die 2–4fache immunologische Sensitivität im Vergleich mit kommerzialisierten Präparaten aufweist, welche kommerziell erhältliches HCG an einen geeigneten Latex gebunden haben.

Beim Verfahren gemäss vorliegender Erfindung wird als Ausgangsmaterial ein relativ unreines HCG verwendet, wie es üblicherweise kommerziell erhältlich ist. Dieses Material enhält HCG in Gegenwart von proteinhaltigem Material. Der Gehalt an HCG in solchen kommerziell erhältlichen Präparaten schwankt gewöhnlich zwischen etwa 2000 und etwa 5000 internationalen Einheiten/mg. Das proteinhaltige Material, welches in solchen Präparaten vorhanden ist, verunreinigt das HCG immunologisch nicht, sondern «verdünnt» es nur. Im Rahmen der vorliegenden Beschreibung werden solche HCG Präparate als «teilweise gereinigtes» HCG bezeichnet.

Im Gegensatz zum vorerwähnten «teilweise gereinigten» HCG versteht der Fachmann unter gereinigtem HCG solches mit ungefähr 10 000 bis ungefähr 20 000 IE/mg. Im Rahmen der vorliegenden Erfindung wurde nun gefunden, dass «teilweise gereinigtes» HCG, d.h. also HCG enthaltend 2000 bis ungefähr 5000 IE/mg, mit einem chaotropischen Mittel behandelt werden kann und dass die daraus isolierte β-Kette – obwohl sie noch in wesentlichem Ausmass proteinhaltiges Material von «teilweise gereinigtem» HCG enthält – zur Herstellung eines äusserst sensitiven Latex-Schwangerschaftsreagenz verwendet werden kann.

Geeignete chaotropische Mittel, welche gemäss vorliegender Erfindung verwendet werden, umfassen eine 7–10molare wässrige Lösung von Harnstoff, eine 7–10molare wässrige Lösung von Lithiumbromid, eine 4,5–6molare wässrige Lösung von Guanidinhydrochlorid und eine 2–3molare wässrige Lösung von Ammoniumthiocyanat. Die Harstofflösung ist bevorzugt.

Die chaotropischen Mittel zur Behandlung des «teilweise gereinigten» HCG gemäss vorliegender Erfindung werden in einem pH-Bereich von 4 bis 5,5, vorzugsweise ungefähr 5, verwendet. Eine anorganische Säure, vorzugsweise Salzsäure, wird zur Einstellung des bevorzugten pH-Bereiches des chaotropischen Mittels verwendet. Für die einzelnen chaotropischen Mittel kann der bevorzugte pH leicht variieren. So z.B. beträgt der bevorzugte pH-Bereich bei Verwendung von Harnstoff als chaotropisches Mittel ungeführ 4,5.

Man lässt das chaotropische Mittel während einer halben bis 2 Stunden, vorzugsweise während ungefähr einer Stunde, in wässriger Lösung auf das «teilweise gereinigte» HCG einwirken. Nach dieser Zeit wird der pH des Reaktionsgemisches auf 7,5 eingestellt. Es wird eine Temperatur von 20–50 °C, vorzugsweise ungefähr 37 °C verwendet. Zum Einstellen des pH der HCG Lösung nach der Auftrennung wird eine geeignete anorganische Base, wie z.B. ein Alkalimetallhydroxyd wie Natriumhydroxyd oder Kaliumhydroxyd verwendet.

Um die Wiedervereinigung der α- und β-Kette von HCG nach der Behandlung mit dem chaotropischen Mittel zu verhindern, wird das Gemisch HCG/chaotropisches Mittel getrennt, und zwar durch Ionenaustausch (Schicht oder Kolonne), Elektrophorese und/oder weiteren herkömmlichen Methoden, gemäss denen Moleküle aufgrund von Differenzen in der elektrostatischen Ladung getrennt werden können. Die Ionenaustauschchromatographie ist bevorzugt. Jedes konventionelle Ionenaustauschmaterial kann zur Trennung der α- und β-Kette von HCG verwendet werden. Ein bevorzugtes Material ist ein hydrophiles, wasserunlösliches, quervernetztes Dextranpolymergel. Dieses Material und die Methode zu seiner Herstellung sind im britischen Patent Nr. 854 715 beschrieben. Das bevorzugte Gelmaterial, welches käuflich von der Firma Pharmacia Fine Chemicals, Uppsala, Schweden unter dem Warenzeichen Sephadex erhältlich ist, besteht aus einem dreidimensionalen, makroskopischen Netzwerk von Dextransubstanzen, welche miteinander verbunden oder quervernetzt sind. Dieses Material ist fähig, unter Schwellen Wasser zu absorbieren. Die Fähigkeit des Gels zur Wasseraufnahme ist umgekehrt proportional zum Grad der Quervernetzung der Dextransubstanzen. Das Gelmaterial ist in einer Vielzahl von Unterschiedlichen Graden bezüglich der Porösität erhältlich. Ähnliche in der Fachwelt bekannte Materialien können ebenfalls verwendet werden. Durch die Verwendung dieser Trennungstechnik erhält man die β-Kette von HCG, welche im wesentlichen frei ist von der verunreinigenden α-Kette. Es wurde gezeigt, dass diese Technik eine Wiedergewinnung von wesentlich mehr als 80% der HCG-Antigeneigenschaften erlaubt. Die «teilweise gereinigte» β-Kette von HCG wird nachher an einen geeigneten immunologisch inerten Latexträger gebunden.

Die Ausdrücke «immunologisch inerte Latexpolymere» oder «immunologisch inerte Latexpolymerträgerpartikel» umfassen wasserunlösliche Latexpolymere mit einer Teilchengrösse von ungefähr 0,01 bis 0,9 µm einem etwa dem von Wasser entsprechenden spezifischen Gewicht. Dies hat zur Folge, dass nach der Bindung der β-Kette von HCG das spezifische Gewicht der Partikel ungefähr dem von Wasser entspricht oder leicht darüber liegt, d.h. 1,01 bis 1,1, wodurch erreicht wird, dass die Teilchen in einer wässrigen Suspension verbleiben. Die Latexpartikel müssen eine genügende Ladungsdichte an der Oberfläche besitzen, damit nach der Bindung mit der β-Kette von HCG ihre abstossenden Kräfte gross genug sind, um eine Agglutination zu verhindern. Die Latexpartikel müssen den immunologischen diagnostischen Tests gegenüber inert sein und vorzugsweise reaktive Gruppen besitzen, welche fähig sind, eine kovalente Bindung mit der β-Kette von HCG zu bilden. Solche Gruppen sind z.B. Carboxygruppen, Aminogruppen oder Gruppen, welche in diese umwandelbar sind. Für die Zwekke der vorliegenden Erfindung besonders geeignete typische Gruppen an den Latexpartikeln sind solche, welche ein aktives Wasserstoffatom besitzen, wie z.B. $-COOH$, $-CONH_2$ oder eine primäre Aminogruppe.

Besonders geeignete Latexträgerteile sind solche, welche in Form einer wässrigen Latexsuspension mit einer Feststoffkonzentration von üblicherweise 50–60% im Handel erhältlich sind. Für die Zwecke der vorliegenden Erfindung eignen sich viele Arten von Latexpolymeren, vorausgesetzt, dass sie die oben erwähnten Kriterien erfüllen. Die vorliegende Erfindung umfasst die Verwendung aller geeigneten Latexpolymere.

Nach der vorliegenden Erfindung wird die β-Kette von HCG mit einem wasserlöslichen Carbodiimid Kopplungsmittel durch eine Amidbrücke kovalent an die Latexpolymerpartikel gebunden.

Beispiele geeigneter Latexpolymere umfassen carboxylenthaltende Polymere und Copolymere wie Polystyrol, Polyvinylpyridin, Styrolbutadien-Copolymere, Vinylacetat/acrylat-Copolymere, Vinylchlorid-acrylat-Copolymere und dergleichen. Die Carboxylgruppen können durch herkömmliche Emulsionspolymerisationstechnik in den Latex eingearbeitet werden oder nachträglich durch Reaktion des bereits hergestellten Latexpolymer oder Copolymer wie dies in der Fachwelt bekannt ist. So z.B. kann ein Monomer wie Acrylsäure, Methacrylsäure, Itaconsäure, Maleinsäure oder dergleichen zum Monomergemisch gegeben werden. Anderseits können Carboxylgruppen auf herkömmliche Weise auf der Oberfläche des Latex gebildet werden, z.B. durch Oxidation von Hydroxylgruppen, Hydrolyse von Acrylat- oder Methacrylatgruppen an der Oberfläche und dergleichen. Die Latices können nach einer Vielzahl von herkömmlichen Emulsionspolimerisationstechniken hergestellt werden, wie z.B. Emulsionspolymerisation im Chargenbetrieb, Emulsionspolymerisation mit Animpfung, halbkontinuierliche oder kontinuierliche Emulsionspolymerisation. Für die Einführung einer Carboxylgruppe ist die Emulsionspolymerisation mit Animpfung bevorzugt, bei der – bevor die Reaktion gestartet wird – ein Impflatex zur Kontrolle der Anzahl der Partikel in das Reaktionsgefäss gegeben wird. In diesem Fall wird ein eine Carboxygruppe enthaltendes Monomer auf der Oberfläche der Impflatexpartikel polymerisiert. Käuflich erhältliche Latices, welche für die Zwecke der vorliegenden Erfindung geeignet sind, umfassen z.B. carboxylierte Styrolbutadien-Copolymere, welche unter den Marken Dow 816 und Dow 421 bei Dow Chemical Company erhältlich sind, und ähnliche.

Als Kopplungsmittel kommt ein wasserlösliches Carbodiimid der allgemeinen Formel

$$R{-}N{=}C{=}N{-}R$$

worin R ein 5 oder 6gliedriger Cycloalkylrest; ein gerader oder verzweigter Alkylrest mit 2–12 Kohlenstoffatomen, wie z.B. Äthyl, n-Propyl, Isopropyl, n-Butyl, sec.-Butyl, tert.-Butyl, Amyl, Hexyl, Octyl, Nonyl, Undecyl, Dodecyl und dergleichen; ein monoarylsubstituierter niederer Alkylrest, wie z.B. Benzyl, α- oder β-Phenyläthyl und dergleichen; ein Monoarylrest wie z.B. Phenyl, Morpholinyl, Piperidyl; ein morpholinylsubstituierter niederer Alkylrest, wie z.B. Morpholinoäthyl; ein di-nieder Alkylaminoniederer alkylrest, und ein pyridylsubstituierter niederer Alkylrest, wie z.B. α, β und γ Methyl- oder Äthylpyridyl bedeutet, sowie Säureadditionssalze und quaternäre Amine davon in Betracht.

Bevorzugte Kopplungsmittel gemäss vorliegender Erfindung sind 1-Cyclohexyl-3-(2-morpholinoäthyl)-carbodiimid metho-p-toluolsulfonat und 1-Äthyl-3-(3-dimethylaminopropyl)-carbodiimid hydrochlorid. Die Reaktion der «teilweise gereinigten» β-Kette von HCG und dem geeigneten Latexträger wird vorzugsweise in Gegenwart des Carbodiimid-Kopplungsmittels in wässrigem Medium, vorzugsweise bei Raumtemperatur (ungefähr 20–25 °C) durchgeführt. Die Temperatur kann jedoch auch zwischen 5 und 40 °C liegen. Um sicher zu stellen, dass die β-Kette von HCG wirksam an den Latexträger gebunden wurde, wird eine Menge an wasserlöslichem Carbodiimid verwendet, welche ungefähr 0,05 bis ungefähr 2 Gew.-% basierend auf dem Gewicht der Trägerpartikel beträgt. Nochmehr bevorzugt wird ungeführ 1 Gew.-% Kopplungsmittel (basierend auf dem Gewicht der Trägerpartikel) verwendet. Der Anteil an Carbodiimid-Kopplungsmittel kann über weite Bereiche variieren, ist jedoch abhängig z.B. von der Anzahl der Carboxylgruppen und der Anwesenheit von zusätzlichen negativ-geladenen Gruppen wie z.B. Sulfonatgruppen auf der Oberfläche der Latexpartikel.

Der pH der Kopplungsreaktion ist wichtig und sollte zwischen 5 und 8, vorzugsweise zwischen ungefähr 6 und 7 liegen. Es ist bevorzugt, den pH der Latexsuspension auf den gewünschten pH-Bereich, d.h. also zwischen ungeführ 6 und 7 zu bringen, bevor er zum Reaktionsgemisch gegeben wird.

Die Reaktion ist nach ungefähr 5 Minuten bis ungefähr 24 Stunden beendet. Im allgemeinen genügen 1½ bis 2½ Stunden. Das erhaltene Produkt ist ein wasserunlösliches Material, welches in einem wässrigen Medium suspendiert ist und einen pH von ungefähr 7 bis 8,5, vorzugsweise 8 aufweist. Das spezifische Gewicht des Materials entspricht ungefähr demjenigen von Wasser, was zur Folge hat, dass die Suspension des Produktes stabil ist. Das Produkt kann z.B. durch Zentrifugation isoliert werden und ist ein weisses, leicht thixotropes, viskoses und lehmartiges Material. Chemisch gesehen besteht das Produkt aus einer ein-molekularen Schicht von «teilweise gereinigter» β-Kette von HCG, welche physikalisch und/oder chemisch über eine Amidbrücke an diskrete Partikel eines immunologisch inerten Trägers gebunden ist. Allfällige vorhandene kontaminierende Proteine beeinflussen serologisch die Empfindsamkeit der β-Kette von HCG nicht, wie dies bereits früher erwähnt wurde.

Für kommerzielle Zwecke wird das gemäss vorliegender Erfindung erhaltene Reagenz in die Form einer wässrigen Lösung gebracht, welche ungefähr 0,5 bis 3,5 Gew.-% der β-Kette von HCG enthält, welche an den immunologisch inerten Träger gebunden ist. Besonders bevorzugt enthalten solche Suspensionen von ungefähr 1 bis ungefähr 2,5 Gew.-% solcher Partikel. Nach einem bevorzugten Aspekt ist die β-Kette von HCG über eine Amidbrücke an carboxyliertes Butadien-Styrol Copolymer gebunden, welches ein Monomerverhältnis von ungefähr 45% Butadien und 55% Styrol aufweist und welches ungefähr 1–5 Gew.-%, gewöhnlich 3 Gew.-% an Carboxylgruppen aufweist.

Die wässrige Suspension der «teilweise gereinigten» β-Kette von HCG, welche an den immunologisch inerten Träger gebunden ist, wird nach der vorliegenden Erfindung vorzugsweise in die Form eines Test-Kits gebracht, welches in einem zweiten Behälter das geeignete Antiserum, d.h. das Anti-HCG-Serum enthält. Das erwähnte Anti-Serum kann in an sich bekannter Weise gewonnen werden. So können Kaninchen oder Ziegen mit hoch-gereinigtem HCG oder gereinigter β-Kette von HCG immunisiert werden, worauf die Antisern gesammelt werden, ihre Titer geprüft werden und dann gelagert werden.

Die nachfolgenden Beispiele illustrieren die Erfindung.

Beispiel 1

100 mg käufliches, rohes HCG, enthaltend ungefähr 2700 IE/mg, werden nach der Methode von Morgan and Canfield in Endocrinology, Vol. 88, p. 1045 (1971) getrennt. Nach dieser Methode wird das rohe HCG in 15 ml einer 10 M wässrigen Lösung von Harnstoff gelöst, worauf der pH der resultierenden Lösung mit Salzsäure auf 4,5 eingestellt wird. Die Lösung wird während einer Stunde bei 37 °C inkubiert, dann werden 3 ml einer 0,03 M wässrigen Lösung von Glycin zugegeben, worauf der pH mit Natriumhydroxid auf 7,5 eingestellt wird.

Eine so erhaltene Lösung enthaltend die getrennte α- und β-Kette von HCG wird der Ionenaustauschchromatographie unterzogen, um die α- und β-Kette physikalisch zu trennen und eine Wiedervereinigung zu vermeiden. Die Chromatographie wird an einer 2,5 × 40 cm Chromatographiesäule durchgeführt, welche bis zu einer Höhe von 20 cm mit Sephadex A-50 (Pharmacia) beschickt ist. Das Produkt hat eine Partikelgrösse (trocken) von ungefähr 40–120 micron, eine Bettvolumen/ml/g des trockenen Gels von 25–33 und eine Kapazität von ungefähr 3 g Hämoglobin/g des Gels. Die Kolonne wird mit einer wässrigen Lösung von Glycin (0,03 M) und Harnstoff (8 M) von pH 7,5 äquilibriert. Die Durchflussgeschwindigkeit wird auf 60 ml/Std. eingestellt, worauf die Lösung enthaltend die α- und β-Kette von HCG auf die Kolonne gebracht wird. Die Säule wird dann mit 500 ml der Glycin/Harnstofflösung eluiert, und das Eluat, welches die α-Kette des HCG enthält, wird auf einmal gesammelt und verworfen.

Die Kolonne wird ein zweites Mal mit 500 ml einer wässrigen Lösung von Glycin (0,2 M), Natriumchlorid (1,0 M) und Harnstoff (8 M) von pH 7,5 eluiert. Die Eluate werden als eine Charge gesammelt, mit einem gleichen Anteil von destilliertem Wasser verdünnt und dann durch Ultrafiltration auf 20 ml verdünnt. Die Fraktion, welche die β-Kette von HCG enthält wird dann mit 0,1 M Natriumboratpuffer vom pH 8,0, welcher 0,02 Gew.-% Natriumazid enthält, äquilibriert.

Durch analytische Scheibenelektrophorese an Proben des ersten und zweiten Eluates konnte gezeigt werden, dass das zweite Eluat die β-Kette von HCG, frei von α-Kette jedoch immer noch Urinproteine enthaltend, enthält, wogegen das erste Eluat α-Kette enthält, kontaminiert mit einem bestimmten Anteil β-Kette HCG und Urinproteinen. Durch Latexagglutination konnte gezeigt werden, dass mehr als 80% HCG Aktivität des rohen Ausgangsmaterials zurückgewonnen werden konnte.

Beispiel 2

Gemäss Beispiel 1 aus «rohem», kommerziell erhältlichem HCG hergestellte β-Kette von HCG wird durch das nachfolgende Verfahren kovalent an immunologisch inerte Latexträgerteilchen gebunden:

5 ml eines Latex aus carboxyliertem Styrolbutadiencopolymer (Dow Nr. 816) werden gewaschen, gemahlen, auf eine Konzentration von ungefähr 78–82 mg/ml eingestellt und dann in ein geeignetes Gefäss mit Rührer gegeben. Unter Rühren wird 1 ml β-Kette von HCG hergestellt nach Beispiel 1 zugegeben und auf eine Konzentration von 3000 IE HCG/ml gebracht.

Wenn die β-Kette von HCG im Latex gut dispergiert ist, werden tropfweise und unter Rühren 3 ml einer 1%igen Lösung von 1-Cyclohexyl-3-(2-morpholinoäthyl)carbodiimid metho-p-toluolsulfonat in destilliertem Wasser zugegeben. Wenn die Zugabe des Kopplungsmittels beendet ist, wird während weiteren 2 Stunden gerührt.

Der gekoppelte Latex wird dann während 30 Minuten bei 5–10 °C bei 1500 rpm zentrifugiert, in welcher Zeit sich der Latex völlig vom Suspensionsmedium abtrennt. Der Überstand wird zur Bestimmung von HCG-Aktivität analysiert und dann verworfen. Der Rückstand wird mit 10 ml destilliertem Wasser gewaschen, wie vorher beschrieben zentrifugiert, worauf der Überstand auf HCG-Aktivität analysiert und verworfen wird. Der Rückstand wird mit 10 ml eines Puffers von pH 8–8,2 gewaschen, welcher 0,1 M Tris-HCl und 0,01% Merthiolat enthält. Der Latex wird erneut in der vorerwähnten Weise zentrifugiert, worauf der Überstand auf HCG-Aktivität untersucht und verworfen wird. Der Rückstand wird wieder mit dem vorerwähnten Puffer gewaschen und dann in 10 ml dieses Puffer resuspendiert. Die Reihenfolge Zentrifugieren, Überprüfung des Überstandes, Waschen und Resuspendieren wird wiederholt, bis zwei aufeinanderfolgende Überstände nach dem Waschen mit Puffer frei von HCG-Aktivität sind. Der gekoppelte, gewaschene Latex ist dann bereit, um in ein diagnostisches Reagenzkit gebracht zu werden und wird in 20 ml des erwähnten Puffers resuspendiert.

Ein typisches Reagenzkit (Reagenz-garnitur)

enthaltend die β-Kette von HCG hergestellt gemäss dem vorstehenden Verfahren würde in einem separaten Behälter Anti-HCG-Serum enthalten. Solche Anti-Seren werden nach herkömmlichen Methoden in Ziegen erzeugt, z.B. durch Immunisieren der Tiere mit hochgereinigtem HCG oder der gereinigten β-Kette von HCG, Sammeln der Antiseren und Überprüfen der Titer. Um Kreuzreaktionen des Anti-HCG mit Urinproteinen zu eliminieren, wird die substraktive Affinitätschromatographie verwendet.

Für dieses Verfahren werden 150 ml gewaschene Sepharose 4 B (Pharmacia) nach der Methode von Primus et al. in J. of Immuno., Vol. 188, p. 55 (1977) aktiviert. 0,5% Urinprotein in 200 ml 0,1 M Natriumboratpuffer vom pH 8,0 werden mit der aktivierten Sepharose 4 B während 18 Stunden bei 4 °C gemischt, um das Protein daran zu kuppeln. Das Produkt wird mit 1 Liter 0,1 M Natriumboratpuffer vom pH 8,0 gewaschen, um ungebundenes Protein zu entfernen, dann mit 500 ml einer 1,5 M wässrigen Lösung von 2-Aminoäthanol vom pH 8,0 gewaschen, um die aktivierten Gruppen des Sepharose 4 B Gels zu reduzieren, worauf mit Natriumboratpuffer vom pH 8,0 re-äquilibriert wird. Das erhaltene Urinproteinadsorbat wird in eine Chromatographiesäule von 4,5 cm Durchmesser gegeben. Zu dieser Kolonne werden nacheinander 10 ml normales Ziegenserum (1 : 2 in 0,1 M Natriumboratpuffer von pH 8,0), 300 ml 0,1 M Natriumboratpuffer vom pH 8,0 und 100 ml 3,0 M Ammoniumthiocyanat in 0,1 M Natriumphosphat vom pH 7,0 gegeben. Die Kolonne wird dann mit 500 ml 0,1 M Natriumboratpuffer re-äquilibriert.

Das Anti-HCG Serum wird mit einem gleichen Anteil 0,1 M Natriumboratpuffer vom pH 8,0 verdünnt und auf die Kolonne gegeben. Zur Kontrolle des Adsorbtionsprozesses wurde ein automatischer Affinitätschromatograph mit Rezyklisierung verwendet. Die verdünnten Antiseren wurden automatisch durch den Apparat gegeben, bis alles chromatographiert war. Eine Probe von 15 ml wurde bei einer Durchflussgeschwindigkeit von 60 ml/Std. chromatographiert. Das Kolonneneluat wurde kontinuierlich durch Adsorbanz kontrolliert und – bevor Protein im Eluat aufschien – wurde der Fluss für 1 Stunde unterbrochen, so dass die Probe mit dem Adsorbanz reagieren konnte. Mit 100 ml 3 M Ammoniumthiocyanat wurde zuerst unadsorbiertes Protein (spezifisches Antiserum zu HCG) und dann adsorbiertes Protein enthaltend Antikörper zu Urinproteinen eluiert. Die unadsorbierten Fraktionen wurden vereinigt und durch Ultrafiltration zum ursprünglichen Volumen der Probe konzentriert.

Durch Latexagglutination wurde gezeigt, dass nach der Adsorption die Wiedergewinnung an Antikörperaktivität mehr als 90% betrug. Die Spezifität der Antiseren wurde durch Immunodiffusion bestimmt und durch Immunoelektrophorese bestätigt.

Beispiel 3

Die Sensitivität der gemäss Beispiel 1 hergestellten β-Kette von HCG wurde wie folgt gezeigt:

Ein Antiserum zu HCG, und zwar Ziegenserum, adsorbiert mit Urinproteinen wurde in einem mit physiologischer Kochsalzlösung gepuffertem Medium titriert. In Gegenwart konstanter Mengen von Latexkonjugaten wurde Anti-HCG verdünnt, und es wurde die letzte Verdünnung bestimmt, bei der nach einer Inkubation von 120 Minuten noch Agglutination oder Flokkulation beobachtet wurde. Die letzte Verdünnung wird als Endpunkt und Titer dieses Antiserums und des korrespondierenden Antigens betrachtet. Die Resultate sind in der nachfolgenden Tabelle I zusammengefasst. Die Latexkonjugate, die verglichen wurden, sind β-Ketten-Latex gemäss Beispiel 2, ein ähnlich hergestelltes α-Ketten-Latex und ein kommerzieller Schwangerschaftstest enthaltend natives HCG gebunden an einen Latex.

Tabelle I

| Verdünnung Anti-HCG | HCG | Latexkonjugat Alpha | Beta |
|---|---|---|---|
| 1:2,000 | 3-4 | 3-4 | 2-3 |
| 1:4,000 | 3-4 | 3 | 3-4 |
| 1:8,000 | 3-4 | neg. | 3-4 |
| 1:16,000 | neg. | neg. | 3-4 |
| 1:32,000 | neg. | neg. | 2 |
| 1:64,000 | neg. | neg. | Spuren |

Eine Bewertung von 3–4 bedeutet starke Reaktion, 3 mittlere Reaktion, 2 mittel bis schwache Reaktion, Spuren sehr schwache Reaktion und neg. keine Reaktion.

Je höher die Verdünnung an Antiserum ist, desto weniger HCG wird zur Neutralisierung desselben benötigt. Die in der obigen Tabelle gegebenen Werte für natives HCG entsprechen ungefähr einer Sensitivität von 1,0 bis 1,25 IE HCG/ml Urin. Die Sensitivität, d.h. die letzte bestimmbare Menge von β-Ketten Latex ist ungefähr 0,25 IE/ml Urin.

Diese Korrelation kann wie folgt gezeigt werden: Von einer Probe von Antiserum wurde bestimmt, mit welcher Menge von HCG in internationalen Einheiten sie reagiert. Eine Reihe von Verdünnungen dieser Probe im oben erwähnten Puffer wurde zu den zu testenden Urinproben gegeben. In jedem Falle wurden 2 ml gepuffertes Antiserum mit 1 ml Urin in einem Teströhrchen vereinigt. 100 Mikroliter des zu testenden Latex wurden zu jeder Probe gegeben, und es wurde durch Umkehren gemischt. Das untere Ende des Teströhrchen wurde während 2 Stunden auf 38 °C erwärmt. Nach dieser Zeit sollte Agglutination oder Flokkulation zu beobachten sein, es sei denn, dass genügend HCG im Urin vorhanden ist, das die Reaktion inhibieren kann. Der Vergleich wurde zwischen einem Schwangerschaftstest enthalten natives HCG und einem β-Ketten produkt gemäss Beispiel 2 durchgeführt. Die Ergebnisse sind in Tabelle II angegeben. Die Anmerkungen haben dieselbe Bedeutung wie für Tabelle I.

Tabelle II

| Konzentration von HCG in der Probe (IE/ml) | HCG | Latexkonjugat Beta |
|---|---|---|
| 0,0 | 3-4 | 3-4 |
| 0,1 | — | 2 |
| 0,2 | — | ½ |
| 0,25 | 3-4 | neg. |
| 0,3 | — | neg. |
| 0,4 | — | neg. |
| 0,5 | 3-4 | neg. |
| 0,75 | 2-3 | |
| 1,0 | 2 | |
| 1,25 | neg. | |

Diese Zahlen zeigen die verbesserte Sensitivität des β-Ketten Latex gemäss Beispiel 2 gegenüber dem Schwangerschaftstest enthaltend natives HCG.

**Patentansprüche**

1. Immunologisches diagnostisches Reagenz zur Bestimmung der Schwangerschaft mittels HCG-Bestimmung und Latex-Agglutination enthaltend diskrete Partikel eines immunologisch inerten Latex-Polymers mit einer Teilchengrösse von 0,01 bis 0,9 µm und einem spezifischen Gewicht von 1,01 bis 1,1, an die Menschliches Choriongonadotropin (HCG) mit einem Carbodiimid-Kopplungsmittel bei einem pH von 5–8 durch eine Amidbrücke kovalent gebunden worden ist, dadurch gekennzeichnet, dass das HCG in Form seiner β-Kette im wesentlichen frei von der α-Kette ist und wobei diese β-Kette durch ½ bis 2stündiges Behandeln von teilweise gereinigtem HCG enthaltend 2000–5000 IE/mg HCG bei einem pH von 4–5,5 und einer Temperatur von 20–50 °C mit einem chaotropischen Mittel, nämlich mit einer 7–10molaren wässrigen Lösung von Harnstoff, einer 7–10molaren wässrigen Lösung von Lithiumbormid, einer 4,5–6molaren wässrigen Lösung von Guanidinhydrochlorid oder einer 2–3molaren wässrigen Lösung von Ammoniumthiocyanat, anschliessende Einstellung des pH des Reaktionsgemisches auf 7,5 und Abtrennung des Gemisches HCG/chaotropisches Mittel durch Ionenaustausch und/oder weitere herkömmliche Methoden, gemäss welchen Moleküle auf Grund von Differenzen in der elektrostatischen Ladung getrennt werden können, von der α-Kette abgetrennt worden ist und wobei weder das teilweise gereinigte HCG noch die β-Kette davon weiter gereinigt worden sind.

2. Reagenz gemäss Anspruch 1, dadurch gekennzeichnet, dass der Latex ein carboxyliertes Styrolbutadiencopolymer ist.

3. Reagenz gemäss Anspruch 1, dadurch gekennzeichnet, dass das wasserlösliche Carbodiimid Kopplungsmittel durch die nachfolgende allgemeine Formel dargestellt wird.

$$R–N=C=N–R$$

worin R Cycloalkyl mit 5–6 Kohlenstoffatomen, gerades oder verzweigtes Alkyl mit 2–12 Kohlenstoffatomen, Monoarylsubstituiertes niederes Alkyl, Monoaryl, Morpholino, Piperidyl, Morpholinosubstituiertes niederes Alkyl, di-nieder-Alkylamino niederes Alkyl oder Pyridyl substituiertes niederes Alkyl bedeutet, Säureadditionssalze davon sowie quaternäre Amine davon.

4. Reagenz gemäss Anspruch 3, dadurch gekennzeichnet, dass das Kopplungsmittel 1-Cyclohexyl-3-(2-morpholinoäthyl)carbodiimid metho-p-toluolsulfonat oder 1-Äthyl-3-(3-dimethylaminopropyl)-carbodiimid Hydrochlorid ist.

5. Verfahren zur Herstellung eines hochempfindlichen immunologischen Diagnosereagenz zur Feststellung der Schwangerschaft mittels HCG-Bestimmung und Latex-Agglutination, bei dem menschliches Choriongonadotropin bei einem pH von 5 bis 8 mit einem Carbodiimid-Kopplungsmittel an diskrete Partikel eines immunologisch inerten Latex-Polymers, die eine Teilchengrösse von 0,01 bis 0,9 µm und ein spezifisches Gewicht von 1,01 bis 1,1 haben, gebunden wird, dadurch gekennzeichnet, dass man das HCG in Form seiner β-Kette einsetzt, die erhalten wird, indem man teilweise gereinigtes HCG enthaltend 2000–5000 IE/mg HCG während ½ bis 2 Stunden bei einem pH von 4–5,5 und bei einer Temperatur von 20–50 °C mit einem chaotropischen Mittel, nämlich mit einer 7–10molaren wässrigen Lösung von Harnstoff, einer 7–10molaren wässrigen Lösung von Lithiumbromid, einer 4,5–6molaren wässrigen Lösung von Guanidinhydrochlorid oder einer 2–3molaren wässrigen Lösung von Ammoniumthiocyanat, behandelt und dabei die Auftrennung in die α-Kette und β-Kette bewirkt, die β-Kette durch Einstellen des pH des Reaktionsgemisches auf 7,5 und Abtrennung des Gemisches HCG/chaotropisches Mittel durch Ionenaustausch und/oder weitere herkömmliche Methoden, gemäss welchen Moleküle auf Grund von Differenzen in der elektrostatischen Ladung getrennt werden können, frei von der α-Kette rückgewinnt, ohne dass man die β-Kette einer weiteren Reinigung unterzieht.

6. Verfahren nach Anspruch 5, dadurch gekennzeichnet, dass der Latex ein carboxyliertes Styrolbutadiencopolymer ist und dass das Kopplungsmittel 1-Cyclohexyl-3-(2-morpholinoäthyl)-carbodiimid metho-p-toluolsulfonat oder 1-Äthyl-3-(3-dimethylaminopropyl)-carbodiimid Hydrochlorid ist.

7. Verfahren zur Bestimmung der Schwangerschaft durch Bestimmen der Anwensenheit von Choriongonadotropin in Körperflüssigkeiten, dadurch gekennzeichnet, dass man eine Probe dieser Flüssigkeit mit Choriongonadotropin-Antiserum mischt und dann diese Mischung mit einer wässrigen Suspension des immunologischen diagnostischen Reagenz gemäss Anspruch 1 kontaktiert und die erhaltenen Resultate beobachtet.

**Claims**

1. An immunological diagnostic reagent for the determination of pregnancy by means of HCG determination and latex agglutination containing discrete particles of an immunologically inert latex polymer having a particle size of 0.01 to 0.9 μm and a specific weight of 0.01 to 1.1, to which human choriongonadotropin (HCG) has been covalently bound via an amide bridge with a carbodiimide coupling agent at a pH of 5–8, characterized in that the HCG is used in the form of its β-chain, whereby this β-chain is substantially free from the α-chain and whereby this β-chain has been separated from the α-chain by treating partially purified HCG containing 2000–5000 IU/mg of HCG for ½ to 2 hours at a pH of 4–5.5 and a temperature of 20–50 °C with a chaotropic agent, namely with a 7–10 molar aqueous solution of urea, a 7–10 molar aqueous solution of lithium bromide, a 4.5–6 molar aqueous solution of guanidine hydrochloride or a 2–3 molar aqueous solution of ammonium thiocyanate, subsequently adjusting the pH of the reaction mixture to 7.5 and separating the mixture of HCG/chaotropic agent by ion exchange and/or further conventional methods in accordance with which molecules can be separated on the basis of differences in the elektrostatic charge, and whereby neither the partially purified HCG nor the β-chain thereof have been purified further.

2. A reagent in accordance with claim 1, characterized in that the latex is a carboxylated styrenebutadiene copolymer.

3. A reagent in accordance with claim 1, characterized in that the water-soluble carbodiimide coupling agent is represented by the following general formula

$$R{-}N=C=N{-}R$$

wherein R signifies cycloalkyl with 5–6 carbon atoms, straight or branched alkyl with 2–12 carbon atoms, monoaryl substituted lower alkyl, monoaryl, morpholino, piperidyl, morpholino-substituted lower alkyl, di-lower alkylamino lower alkyl or pyridyl-substituted lower alkyl, acid addition salts thereof as well as quaternary amines thereof.

4. A reagent in accordance with claim 3, characterized in that the coupling agent is 1-cyclohexyl-3-(2-morpholinoethyl)carbodiimide metho-p-toluenesulphonate or 1-ethyl-3-(3-dimethylaminopropyl)-carbodiimide hydrochloride.

5. A process for the manufacture of a highly sensitive immunological diagnostic reagent for the determination of pregnancy by means of HCG determination and latex agglutination, in which human choriongonadotropin is bound at a pH of 5 to 8 with a carbodiimide coupling agent to discrete particles of an immunologically inert latex polymer which have a particle size of 0.01 to 0.9 μm and a specific weight of 1.01 to 1.1, characterized in that the HCG is used in the form of its β-chain which is obtained by treating partially purified HCG containing 2000–5000 IU/mg of HCG for ½ to 2 hours at a pH of 4–5.5 and at a temperature of 20–50 °C with a chaotropic agent, namely with a 7–10 molar aqueous solution of urea, a 7–10 molar aqueous solution of lithium bromide, a 4,5–6 molar aqueous solution of guanidine hydrochloride or a 2–3 molar aqueous solution of ammonium thiocyanate and thereby bringing about the separation into the α-chain and β-chain, and recovering the β-chain free from the α-chain by adjusting the pH of the reaction mixture to 7.5 and separating the mixture of HCG/chaotropic agent by ion exchange and/or further conventional methods in accordance with which molecules can be separated on the basis of differences in the electrostatic charge, without the β-chain being subjected to a further purification.

6. A process according to claim 5, characterized in that the latex is a carboxylated styrene-butadiene copolymer and in that the coupling agent is 1-cyclohexyl-(2-morpholinoethyl)-carbodiimide metho-p-toluenesulphonate or 1-ethyl-3-(3-dimethylaminopropyl)-carbodiimide hydrochloride.

7. A method for the determination of pregnancy by determining the presence of choriongonadotropin in body fluids, characterized in that a sample of this fluid is mixed with choriongonadotropin antiserum and this mixture is then contacted with an aqueous suspension of the immunological diagnostic reagent in accordance with claim 1 and the results obtained are observed.

**Revendications**

1. Réactif diagnostique immunologique pour le détermination de la grossesse par détermination de l'HCG et de l'agglutination du latex contenant des particules discrètes d'un polymètre de latex immunologiquement inerte d'une granulométrie de 0,01 à 0,9 μm et d'un poids spécifique de 1,01 à 1,1, à la gonadotropine chorionique humaine (HCG) avec un agent de couplage carbodiimide à un pH de 5–8 lié de façon covalente par un pont amide, caractérisé en ce que l'HCG est utilisée sous la forme de sa chaîne β, où cette chaîne β est pour l'essentiel dépourvue de chaîne α et où cette chaîne β est séparée de la chaîne α par traitement pendant ½ à 2 h d'HCG partiellement purifié contenant 2000–5000 IE/mg d'HCG à un pH de 4–5,5 et à une température de 20–50 °C avec un agent chaotropique, à savoir avec une solution aqueuse 7–10 molaire d'urée, une solution aqueuse 7–10 molaire de bromure et lithium, une solution aqueuse 4,5–6 molaire de chlorhydrate de guanidine ou une solution aqueuse 2–3 molaire de thiocyanate d'ammonium, puis où l'on amène le pH du mélange réactionnel à 7,5 et où l'on sépare le mélange HCG/agent chaotropique par échange d'ions et/ou par d'autres procédés habituels, selon lesquels on peut séparer les molécules sur la base des différences de charge électrostatique, et où ni l'HCG partiellement purifiée, ni sa chaîne β ne sont purifiées plus avant.

2. Réactif selon la revendication 1, caractérisé en ce que le latex est un copolymère styrène-butadiène carboxylé.

3. Réactif selon la revendication 1, caractérisé en ce que l'agent de couplage carbodiimide soluble dans l'eau est représenté par la formule générale suivante:

$$R-N=C=N-R$$

où R représente un cycloalcoyle en $C_5$ à $C_6$, un alcoyle en $C_2$ à $C_{12}$ à chaîne droite ou ramifiée, un alcoyle inférieur substitué par un monoaryle, un monoaryle, un morpholino, un pipéridyle, un alcoyle inférieur substitué par un morpholino, un di-alcoyle inférieur-amino-alcoyle inférieur ou un alcoyle inférieur subsitué par un pyridyle, ses sels d'addition d'acides ainsi que ses amines quaternaires.

4. Réactif selon la revendication 3, caractérisé en ce que l'agent de couplage est la 1-cyclohexyl-3-(2-morpholinoéthyl)carbodiimide-métho-p-toluènesulfonate ou le chlorhydrate de 1-éthyl-3-(3-diméthylaminopropyl)-carbodiimide.

5. Procédé de préparation d'un réactif diagnostic immunologique hautement sensible pour la détermination de la grossesse par détermination de l'HCG et agglutination du latex, où on lie la gonadotropine chorionique humaine à un pH de 5 à 8 avec un agent de couplage carbodiimide à des particules discrètes d'un polymètre de latex immunologiquement inerte qui ont une granulométrie de 0,01 à 0,9 µm et un poids spécifique de 1,01 à 1,1, caractérisé en ce qu'on utilise l'HCG sous la forme de sa chaîne β que l'on obtient en traitant de l'HCG partiellement purifié contenant 2000–5000 IE/mg d'HCG pendant ½ à 2 h à un pH de 4–5,5 et à une température de 20–50 °C avec un agent chaotropique, à savoir avec une solution aqueuse 7–10 molaire d'urée, une solution aqueuse 7–10 molaire de bromure de lithium, une solution aqueuse 4,5–6 molaire de chlorhydrate de guanidine ou une solution aqueuse 2–3 molaire de thiocyanate d'ammonium et en provoquant ainsi la séparation en la chaîne α et la chaîne β, en récupérant la chaîne β en la libérant de la chaîne α par réglage du pH du mélange réactionnel à 7,5 et séparation du mélange HCG/agent chaotropique par échange d'ions et/ou par d'autres procédés habituels selon lesquels on peut séparer les molécules sur la base des différences de charge électrostatique, sans soumettre la chaîne β à une purification plus poussée.

6. Procédé selon la revendication 5, caractérisé en ce que le latex est un copolymère styrène-butadiène carboxylé et en ce que l'agent de couplage est le 1-cyclohexyl-3-(2-morpholinoéthyl)-carbodiimide-métho-p-toluènesulfonate ou le chlorphydrate de 1-éthyl-3-(3-diméthylaminopropyl)-carbodiimide.

7. Procédé de détermination de la grossesse par détermination de la présence de gonadotropine chorionique dans les liquides corporels, caractérisé en ce qu'on mélange un échantillon de ce liquide avec de l'antisérum antigonadotropine chorionique puis en ce qu'on met ce mélange en contact avec une suspension aqueuse du réactif diagnostique immunologique selon la revendication 1 et qu'on en observe les résultats obtenus.